Europäisches Patentamt

**European Patent Office**

**Office européen des brevets**

(11) Publication number: **0 207 758**
**A2**

## (12) EUROPEAN PATENT APPLICATION

(21) Application number: **86305011.8**

(22) Date of filing: **27.06.86**

(51) Int. Cl.⁴: **A 61 F 5/00**

(30) Priority: **01.07.85 GB 8516582**

(43) Date of publication of application:
**07.01.87 Bulletin 87/2**

(84) Designated Contracting States:
**AT BE CH DE FR IT LI LU NL SE**

(71) Applicant: **UNIVERSITY COLLEGE LONDON
Gower Street
London WC1E 6BT(GB)**

(72) Inventor: **Smith, David Martin
20 Woodstock Way Mitcham
Surrey CR4 1BA(GB)**

(74) Representative: **Charlton, Peter John et al,
Elkington and Fife High Holborn House 52/54 High
Holborn
London WC1V 6SH(GB)**

(54) Method for forming prostheses or orthoses.

(57) The present invention relates to a method for forming prostheses or orthoses, wherein from data on the dimensions of the relevant portion of patient a plurality of individual slices are carved, the slices subsequently being brought into registration to form a positive model of the relevant portion. In the case of a leg prostheses, for example, the model can be used as a mould around which to form the stump socket, optionally by vacuum moulding. Also the remaining parts of the blanks can be registered to form a container for the relevant portion, the container being subsequently used as a temporary prosthesis or orthosis. The remaining parts can be further carved to give as cosmetic shape to the container.

## METHOD FOR FORMING PROSTHESES OR ORTHOSES

The present invention relates to a method for forming prostheses or orthoses.

Conventionally, prostheses ororthoses are formed by vacuum forming a socket, for example, over a mould of the relevant portion or a patient which has been formed by carving a solid block to match the shape of that portion. The carving operation may be automated and controlled via a microprocessor into which the dimensions of the portion of the patient have been fed.

According to the invention there is provided a method for forming prostheses or orthoses, wherein from data on the dimensions of the relevant portion of patient a plurality of individual slices are carved, the slices subsequently being brought into registration to form a positive model of the relevant portion.

By "relevant portion" of the patient is meant the stump in the case of a prosthesis or, for example, the arm in the case of an arm orthosis.

In the case of a leg prosthesis, for example, the model can be used as a mould around which to form the stump socket, optionally by vacuum moulding.

The invention also provides a method for forming prostheses or orthoses, wherein from data on the dimensions of the relevant portion of the patient a plurality of individual slices are carved from blanks, these slices being registrable to form a model of the relevant portion, and wherein the remaining parts of the blanks are registered to form a container for the relevant portion, the container being subsequently used as a temporary prosthesis or orthosis or a mould in which a cast of the portion can be made.

Preferably the slices are themselves also registered to form a positive model of the relevant portion.

The container or "negative" model can be fitted on the patient and any uncomfortable points are related to the positive model and slices of the negative or positive model can be recarved. To facilitate the comparison between the negative model when fitted and the positive model, every $n^{th}$ slice could be coloured. If the negative model is comfortable, the positive model can be used to form the final prosthesis or orthosis.

In this method, a surprising and unexpectedly advantageous use of the "discard" left after the forming of the positive model or mould is achieved.

The invention further provides a method for forming prostheses or orthoses, wherein from data on the dimensions of the relevant portion of the patient a plurality of indivudual slices are carved from blanks, these slices being registrable to form a model of the relevant portion, wherein the remaining parts of the blanks can be registered to form a negative

model of the relevant portion, and wherein the remaining parts are also carved to give an external cosmetic shape to the negative model when the parts are registered.

Preferably, the further carving takes place at the same time as the initial carving of each blank, i.e. before the blanks are registered.

The cosmetic shape may be predetermined according to each individual patient, e.g. to match the other limb of the patient in the case of prosthesis. Alternatively, the outer cosmetic shape of the negative model may be a standard shape for all patients or the outer shape may be determined by simply cutting a fixed width around the first carving. In this case, the negative model will have a constant thickness to its walls.

It should be noted that if the instructions to the carver are coming from the same source, the outer shape could be cut either before or after inner shape.

The negative model having the outer cosmetic shape can be used directly as the prosthesis or orthosis itself if the blanks are made of a suitable structural material or if the negative model is provided with a suitable structural coating or reinforcement.

It is clear that this method has great advantages over known methods of forming prosthesis or orthosis since in the one operation of forming the "mould" the final prosthesis or orthosis is simultaneously formed.

All of the methods have the advantage of limiting the number of visits necessary by the patient to the limb-fitting centre since a final comfortable shape can be achieved in a relatively short period of time.

Moreover, given the necessary hardware, the limb fitting centres could be mobile and thus actually visit the homes of the patients because of the simplicity of the methods. The hardware is a simple carving machine and a microprocessor which instructs the carver and into which the dimensions of the patient are initially fed.

The data regarding the dimensions of the patient can be processed as explained in the publication "Computer Aided Design of Prosthetic Sockets for Below Knee Amputees", C.G. Saunders et al, Prosthetics and Orthotics International, Apr 1985, Vol 9, No. 1, P17-22. This data, stored in the microprosesor or computer, can be used to instruct the carver to carve each slice to the correct shape and angle the walls of each slice to fit with adjacent slices.

Embodiments of the present invention are described below in more detail, by example only, and with reference to the accompanying drawings wherein:

Fig. 1 shows, in a perspective partly cut-away view, a carving machine for performing the methods of the invention;

Fig. 2 shows in section the slices registered to form a negative model and positive model;

Fig. 3 shows one example of a slice formed according to the invention;

Fig. 4 shows one example of the registration of individual slices; and

fig. 5 shows a detail of one prosthesis made according to the invention.

To perform the methods of the invention, a control unit, namely a computer or micro-processor, is needed in which a data-bank with the dimensions of the relevant portions of the patient can be stored or a digital model of the relevant portion is stored. A realisation of the data-bank or digital model is described in the above mentioned publication. The other piece of hardware needed is the carver which cuts each individual slice.

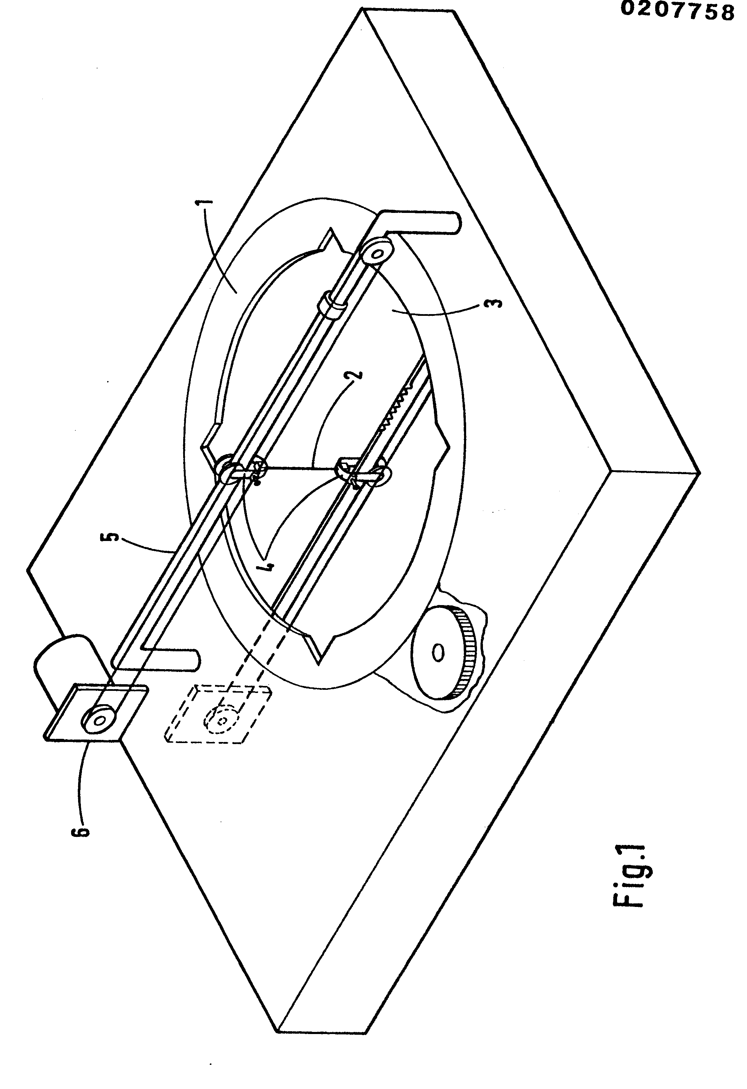

An example of the suitable carving machine is shown in Fig. 1. The control unit is connected to the carver and instructs the carving machine on the basis of the digital model stored in the control unit. The programming of the control unit is conventional, e.g. control algorithm performs linear interpolations to transfer each axis between the given data points, as is known in the art.

The carver comprises a rotatable turntable 1, on which each slice sits, and a movable hot wire 2 which traverses the turntable and thus can cut the blank as desired. The control unit controls the rotation of the turntable and the angle and position of the hot wire.

The turntable 1 is annular in shape with a central aperture 3 within which the hot wire can move. The hot wire is suspended on supports 4 between two rods 5 parallel to the plane of the turntable, one below and one above. The supports which are mounted on the rods are driven along the rods by means of a "pulley" arrangement 6, the pulley wheels at one end of the rod being driven by stepper motors.

The turntable is also driven by a stepper motor, which engages the edge of the turntable. The stepper motor can operate at 1000 steps/s so that each revolution of the turntable, i.e. each carving of one slice, can take 10s with a gear ratio of 10:1. The turntable has four corner sections cut from the inside ring in which the four corners of a square of rectangular blank can be positioned.

The power source and connections of the hot wire are not illustrated in detail but are conventional.

The carver is suitable for expanded polystyrene blanks and other materials which can be cut by heat.

The dirves for the hot wire are controllable by the control unit, as is the turntable. The upper and lower mounts are independently driven so that the angle of the wire can be adjusted as required. The turntable is rotated to move the blank tile relative to the hot wire.

Obviously, the hot wire must begin at the edge of the tile and cut inwards, whereafter it cuts a central hole in tile and then exits through the same cut. In practice, the wire should enter from a different side of each blank so that when the individual slices are registered there is no single line of weakness along the length of the mould.

Each blank is cut in turn and then is removed from the carver and registered with the previous blanks. The operation of putting tiles on the turntable and removing them can of course be automated. The tiles may be glued together or, in an automatic operation, could be heat welded automatically as they are transferred from the turntable onto the stack of earlier tiles cut.

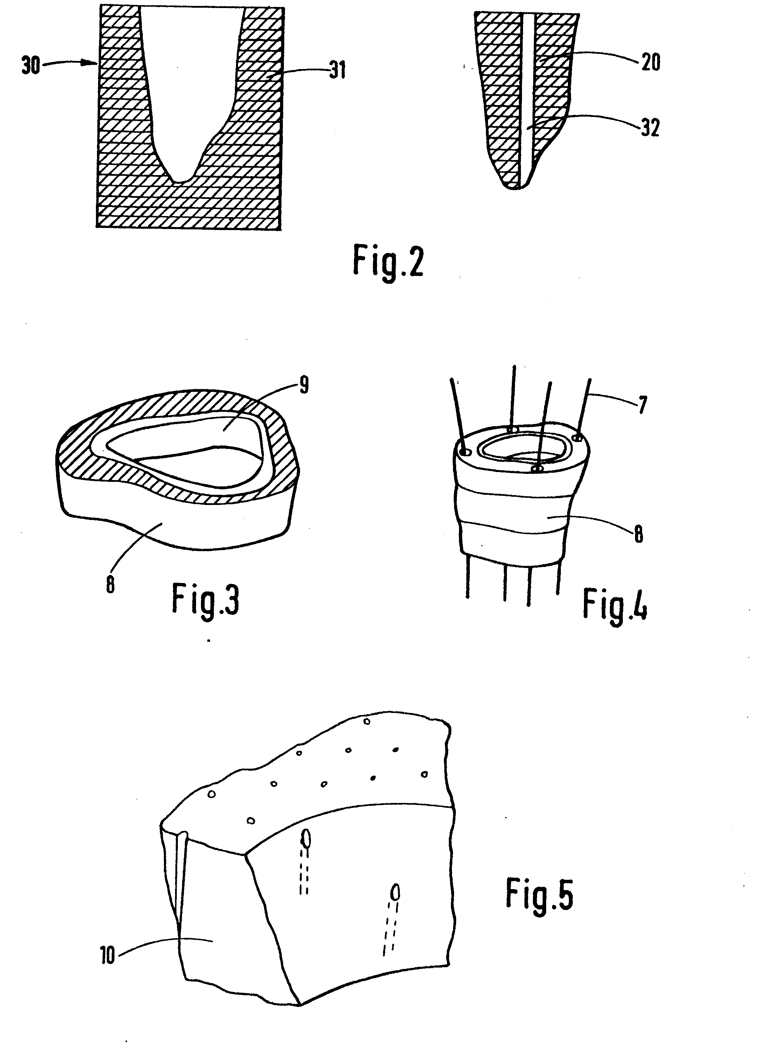

Where only an internal cut is made to form a positive mould 20 and negative mould 30, the negative mould 30 may easily be registered because of the external shape of the blank 31 is always the same, generally square, as seen in fig. 2. For the positive mould, it is preferable for a central hole 32 to be initally cut in the blank in the shape of a triangle or square, for example, i.e. any shape which will prohibit relative rotation of the slices. The slices 33 of the postive mould are then registered by mounting the slices on a central rod of a corresponding triangular or square cross-section.

Where an external cosmetic shape is given to each blank in the case of the negative mould, the tiles may be registered by means of mating lugs or radial ridges, or by cutting small holes through the wall of each slice and then passing carbon fibres 7 through the holes running the length of the orthosis or prosthesis (this is illustrated in Fig. 4). The slices can thus be pulled into registration. In this case, if the fibres are prestressed, no gluing may be necessary.

In the simplest method of the invention, the positive model formed by a number of registered slices is simply used as a mould around which the stump socket, for example, is formed by conventional processes, e.g. vacuum moulding.

In another form of the invention, the outer part of each slice is not discarded but is used to form the negative model of the patient's stump, for example. This negative model may, as mentioned above, be used as a temporary prosthesis and can be fitted on the patient to test for comfort. If each fifth slice of the models is a different colour, then the patient's reaction to the comfort of different areas of the temprary prosthesis can easily be related to the positive model and the location and amount of adjustment to the model can be decided by the prosthetist. The incorrect slices of both models can be recarved until the patient and prosthetist are satisfied. The negative or positive model can then be used to derive the socket. Plaster could be poured in the negative mould to form a robust positive model. If the sliced positive mould is actually used to form the socket, it may be dipped in resin or the like to give it a sufficiently hard coating to accept the moulding operation.

Where an outer cosmetic shape is also cut from the blank then, if the material is of a suitable structural density, what is formed is the final prosthesis, the inner shape of the stack of registered slices corresponding to the patients stump when optionally loaded, the outer shape being of a suitable cosmetic appearance. The surfaces of the prosthesis or orthosis then simply needs to be treated, by resin for example, and fitted to another prosthetic element, e.g. a shin tube.

Also, if an outer cosmetic shape is carved, the outerportions of the carved blanks can be registered, forming a negative model of the cosmetic shape. If the positive model of the stump is aligned within this negative model and structural foam is injected into the gap, then a prostheticmember is directly produced.

As illustrated in fig. 3 each slice may be formed of two "concentric" rings by which the functions of global load transfer and local comfort through compliance are separated. The outer ring 8 is of hard foam, the inner ring 9 of soft liner having a thickness proportional to the desired compliance. The final assembly is resin dipped.

In Fig. 5 is seen a detail of a prosthesis in which the wall of the ring 10 is porous and so, if a porous liner is used, the socket will be able to breathe. Thus the properties of the material of the blanks can contribute to the comfort of the socket in a way which is not possible with conventional methods.

Of course, in the case of a leg prosthesis, all of the slices may not be hollow, so the lower slices which do not need to accommodate the patients stump can be cut to conform to the lower leg shape, including if necessary the foot. Thus, by the method of the invention a complete prosthesis (or orthosis) can be formed in one simple operation.

If necessary, different blanks can be formed of materials having different physical properties, for example flexibility, in which case a prosthesis having limited amounts of movement at particular points can be obtained.

It will be appreciated from the above that where an outer cosmetic shape is formed, orthoses can also be easily made, for example back braces, arm splints, insoles or orthopaedic shoes. The ring of each blank can be registered with the other rings and this forms a tube which will precisely match the patients back nor arm etc. or may apply desired corrective forces.

CLAIMS

1.    A mehtod of forming prostheses or orthoses, comprising the steps of:

a)    determining the dimensions of a relevant portion of a patient;

b)    on the basis of the said dimensions,  carving a slice which corresponds to one cross-section of predetermined thickness of the said portion;

c)    repeating step b); and

d)    registering the carved slices to form a positive model of the said portion.


2.    A method according to claim 1, comprising the further step of moulding a socket around the positive model.


3.    A method according to claim 2, wherein the socket is moulded by vacuum moulding.


4.    A method according to claim 1, wherein the blanks are carved again to provide a cosmetic outer shape, wherein the outer portions of the blanks are registered to form a negative model of the cosmetic shape, wherein the said positive model and the said negative model are aligned and the space  between them filled to form an orthosis or prosthesis.

5.    A method according to claim 1 wherein all the slices are also carved with a central shape to facilitate registration on a member of that shape.

6.    A method of forming prostheses or orthoses, comprising the steps of:

a)    determining the dimensions of a relevant portion of a patient;

b)    on the basis of the said dimensions, carving from a blank a slice which corresponds to one cross-section of predetermined thickness of the said portion;

c)    repeating step b); and

d)    registering the carved blanks to form a negative model of the said portion.

7.    A method according to claim 6, comprising the additional step of also registering the carved slices to form a positive model of the said portion.

8.    A method according to claim 7, wherein every $n^{th}$ blank is distinguishable from the other blanks.

9.    A method of forming prostheses or orthoses, comprising the steps of:

a)    determining the dimensions of a relevant portion of a patient;

b)      on the basis of the said dimensions, carving
from a blank a slice which corresponds to one cross-
section of predetermined thickness of the said portion;

c)      carving the said blank again to provide a cosmetic
outer shape;

d)      repeating steps b and c; and

d)      registering the twice-carved blanks to form a
negative model of the said portion having a cosmetic
shape.

10.      A method according to claim    , comprising
the further step of carving and registering
slices of a predetermined size, for example in the
shape of a foot.

Fig.1

Fig.2

Fig.3

Fig.4

Fig.5